## Europäisches Patentamt
## European Patent Office
## Office européen des brevets

(19)

(11) Numéro de publication: **0 148 048**

**A1**

(12) # DEMANDE DE BREVET EUROPEEN

(21) Numéro de dépôt: 84402377.0

(22) Date de dépôt: 21.11.84

(51) Int. Cl.⁴: **C 07 C 1/04**
B 01 J 29/06, B 01 J 23/74

(30) Priorité: 07.12.83 FR 8319591

(43) Date de publication de la demande:
10.07.85 Bulletin 85/28

(84) Etats contractants désignés:
BE DE GB IT LU NL SE

(71) Demandeur: COMPAGNIE FRANCAISE DE RAFFINAGE
Société anonyme dite:
5, rue Michel-Ange
F-75781 Paris Cedex 16(FR)

(72) Inventeur: Jacobs, A. Peter
Strealand 104
B-1686 Gooik(BE)

(72) Inventeur: Willemen, Karl
Donkse Steenweg 217
B-2130 Brasschaat-Antwerpen(BE)

(72) Inventeur: Leplat, Lieve
Synt Servaas Straat 8
B-3041 Pellenbirg(BE)

(72) Inventeur: Uytterhoeven, Jean-Baptiste
Rotspoel Straat 361
B-3030 Leuven Heverlee(BE)

(72) Inventeur: Marty, Claude
66, rue Guillemard
F-76600 Le Havre(FR)

(72) Inventeur: Cormerais, Francois-Xavier
110, Boulevard Albert Ier
76600 Le Havre(FR)

(72) Inventeur: Engelhard, Philippe A.
197, rue Félix Faure
F-76600 Le Havre(FR)

(74) Mandataire: Brot, Philippe
CABINET BROT et JOLLY 83, rue d'Amsterdam
F-75008 Paris(FR)

(54) Composition catalytique et application à la conversion de gaz de synthèse en un mélange d'hydrocarbures à aromaticité élevée.

(57) L'invention concerne une nouvelle composition catalytique comprenant un mélange intime de poudres d'au moins un composé à base de fer ou de cobalt actif dans la synthèse de Fischer-Tropsch et d'une zéolithe.

Selon l'invention, ledit composé repose sur un support.

L'invention concerne également l'application de cette composition à la conversion de gaz de synthèse en un mélange d'hydrocarbures à aromaticité élevée.

## Nouvelle composition catalytique et application à la conversion de gaz de synthèse en un mélange d'hydrocarbures à aromaticité élevée.

La présente invention concerne la conversion de gaz de synthèse, c'est-à-dire de mélanges d'oxydes de carbone, d'une part, et d'hydrogène ou de composés aptes à produire de l'hydrogène, d'autre part, en mélanges d'hydrocarbures. Elle concerne plus particulièrement une nouvelle composition catalytique et son application à la conversion de tels gaz de synthèse en mélanges d'hydrocarbures à aromaticité élevée, c'est-à-dire à teneur élevée en hydrocarbures aromatiques.

De nombreux procédés de conversion de carbone ou d'hydrocarbures en mélanges gazeux plus ou moins complexes, comprenant essentiellement de l'hydrogène et du monoxyde et/ou dioxyde de carbone, ont déjà été proposés dans la technique.

Par ailleurs, il est également connu de convertir catalytiquement les gaz de synthèse ainsi obtenus en hydrocarbures, notamment liquides, susceptibles d'applications importantes, et en dérivés oxygénés.

Parmi les procédés proposés, l'un des plus connus, du fait des applications pratiques qu'il a connues, est le procédé dit Fischer-Tropsch, qui utilise comme catalyseurs des métaux ou des oxydes des métaux des Groupes IB, IIB, IIIB, IVB, VIB et VIII, seuls ou en mélange. Parmi ces métaux, on mentionnera notamment le zinc, le fer, le nickel, le cobalt, le thorium, l'osmium, le ruthénium, le rhodium, etc..., éventuellement en combinaison avec des promoteurs tels que le cuivre, le fer, l'alumine, l'oxyde de chrome, des composés alcalins ou alcalino-terreux, des terres rares, etc...

Ces catalyseurs du procédé Fischer-Tropsch sont parfois mélangés à des matériaux de remplissage (en anglo-saxon "f_ll( ), tels que le Kieselguhr, et les avantages de ce mélange ont été notamment décrits, mais ces matériaux de remplissage ne sont pas à proprement parler des supports, car ils n'ont pas de surface et ne retiennent donc pas les atomes actifs.

Dans leur application à la production d'hydrocarbures de la coupe essence à partir de gaz de synthèse, ces catalyseurs présentent cependant divers inconvénients. Leur sélectivité, en particulier, est médiocre, puisqu'ils conduisent à des coupes d'hydrocarbures de 1 à 40 atomes de carbone. De plus, les hydrocarbures formés sont essentiellement linéaires (n-paraffines et n-oléfines), avec pour conséquence un mauvais indice d'octane de la coupe essence et la nécessité de traitements complémentaires.

Pour remédier à ces inconvénients, il a été envisagé plus récemment de combiner les catalyseurs classiques de Fischer-Tropsch avec des catalyseurs zéolithiques. En effet, du fait de leurs propriétés structurales, les zéolithes inhibent la formation de trop grosses molécules hydrocarbonées, qui ne peuvent pas se former à l'intérieur du réseau cristallin. Par ailleurs, la forte acidité de ces catalyseurs leur confère des propriétés d'aromatisation, de craquage et d'isomérisation des grosses molécules linéaires.

C'est à ce type de réaction catalytique que s'intéresse la présente invention et, plus particulièrement, aux procédés dans lesquels le composé actif dans la synthèse de Fischer-Tropsch, utilisé en combinaison avec une zéolithe, est une poudre à base de fer ou de cobalt.

Il a été proposé, en effet, de mélanger intimement en un seul lit des composés actifs dans la synthèse Fischer-Tropsch et des zéolithes (voir le brevet belge n° 828 228 et le brevet U.S. n° 4 086 262). Les deux types de catalyseurs ont toutefois des

3    **0148048**

conditions d'utilisation optimale différentes et, en vue d'obtenir un rendement et une sélectivité maximum en coupe essence, il est nécessaire de choisir avec soin la température et la pression de la réaction, le rapport $H_2/CO$ du gaz et synthèse traité et la vitesse spatiale horaire de celui-ci (brevet U.S. n°4 157 338). De plus, la Demanderesse a constaté que, lorsqu'on utilise dans ce cas une poudre à base de fer en mélange avec une zéolithe, l'activité aromatisante de la zéolithe diminue rapidement et devient faible en comparaison de ce qu'elle est en l'absence de fer. Il semblerait que les atomes de fer migrent à l'intérieur du réseau cristallin de la zéolithe, ce qui aurait pour effet de bloquer irréversiblement les sites acides de cette zéolithe, dont la fonction aromatisante est prépondérante, et par conséquent d'empêcher la transformation de la charge à traiter.

C'est ce que confirment apparemment d'autres essais visant à introduire directement dans la zéolithe le composé actif en synthèse de Fischer-Tropsch ("The role of metal zeolite interaction in indirect liquefaction catalysis", V.U.S. RAO, Conference on Surface Science, Tampere, Finlande, 18-20 Août 1982). Il ressort en effet de ces travaux que, dans le cas de fer pulvérulent, il est important qu'il soit finement dispersé sur la surface de la zéolithe, mais qu'il pénètre le moins possible dans ses pores.

Pour éviter ces problèmes d'"empoisonnement" des sites acides de la zéolithe par les atomes de fer, on utilise donc généralement, dans ce cas, deux lits catalytiques séparés (brevets U.S. nos. 4 159 995, 4 304 871 et 4 279 830 ; brevets européens nos. 20 140 et 20 141). Ce processus assure un rendement satisfaisant en composés aromatiques et se prête à une régénération séparée des deux catalyseurs. Du fait de l'emploi de deux réacteurs distincts, il est toutefois coûteux et délicat à mettre en oeuvre.

La présente invention vise à remédier à ces inconvénients en proposant un système catalytique comprenant, en combinaison, un catalyseur du procédé Fischer-Tropsch à base de fer ou de cobalt et une zéolithe, ou tout autre métallo-silicate cristallin microporeux, qui puisse être utilisé en un seul lit pour la conversion d'un gaz de synthèse en un mélange d'hydrocarbures à aromaticité élevée, sans que l'activité aromatisante de la zéolithe soit altérée par la présence de ces matières actives, celles-ci étant immobilisées sur le support.

L'invention vise également à proposer un procédé utilisant un tel système catalytique pour la conversion d'un gaz de synthèse en un mélange d'hydrocarbures à aromaticité élevée, dans lequel la teneur en hydrocarbures aromatiques du mélange obtenu ne diminue pas au cours du temps.

A cet effet, l'invention a pour objet une composition catalytique comprenant un mélange intime de poudres d'au moins un composé à base de fer ou de cobalt actif dans la synthèse de Fischer-Tropsch et d'une zéolithe, cette composition étant caractérisée en ce que ledit composé repose sur un support, par exemple constitué d'alumine, de silice, de zéolithe, d'argile intercalée, ou plus généralement d'oxydes de métaux ou d'un mélange de plusieurs ou de tous ces composés.

Le support utilisé pour le composé aura avantageusement des pores d'une dimension moyenne comprise entre 3 $\overset{\circ}{A}$ et 20 000 $\overset{\circ}{A}$ et, de préférence, entre 5 $\overset{\circ}{A}$ et 5 000 $\overset{\circ}{A}$.

Le composé pourra être tout composé à base de fer ou de cobalt actif dans la synthèse de Fischer-Tropsch, notamment sous la forme métallique ou oxyde, seul ou en mélange, éventuellement en présence de promoteurs, notamment de composés alcalins tels que les carbonates, notamment de potassium. L'addition d'oxyde de potassium

accroît l'activité de la composition catalytique et favorise la formation de produits plus lourds, dans le cas de son application à la conversion des gaz de synthèse.

Comme zéolithe, on utilisera avantageusement, dans la composition catalytique conforme à l'invention, des zéolithes sous la forme acide, et présentant une bonne stabilité dans la conversion des produits primaires de la réaction de Fischer-Tropsch, ou toute autre substance équivalente telle que des composés métallo-silicate cristallins microporeux. Un exemple non limitatif des zéolithes préférées pour cette combinaison est constitué par la série des zéolithes du type ZSM-5, ZSM-11, ZSM-12, ZSM-34, ZSM-35, ZSM-38, ZSM-48, silicalite, zéolithe PHI, zéolithe BETA, les zéolithes du type ZSM-5 étant préférées.

La quantité de fer, d'oxyde de fer ou de composé à base de fer déposée sur ce support sera avantageusement comprise entre 1 et 25 % en poids du catalyseur supporté.

Dans la composition catalytique conforme à l'invention, la zéolithe représentera entre 5 et 98 % en poids de cette composition et, de préférence, entre 40 et 95 %.

Un autre objet de l'invention est constitué par l'application de la composition catalytique définie ci-dessus à la conversion de gaz de synthèse en un mélange d'hydrocarbures à aromaticité élevée, par passage dudit gaz de synthèse, dans des conditions de conversion, sur un lit de ladite composition catalytique.

Les conditions de mise en oeuvre de ce procédé peuvent être les suivantes :

- température                        :     20 à 550°C,
- pression                           :      1 à 100 bar,
- vitesse spatiale horaire           :    100 à 10 000 v/v/h,
- rapport $H_2CO$ de la charge traitée :     0,5 à 3,5

De préférence, afin d'accroître la teneur en hydrocarbures aromatiques de la coupe essence du mélange d'hydrocarbures obtenu, le composé supporté de la composition catalytique comprendra au moins un promoteur choisi dans le groupe suivant : le cuivre, le fer, le cobalt, l'oxyde de chrome, des composés alcalins ou alcalino-terreux, le ruthénium et d'autres métaux du groupe VIII, des terres rares, généralement sous la forme d'oxydes, sans que ceci puisse être considéré comme une quelconque limitation.

Ainsi qu'il a été indiqué ci-dessus, ce promoteur pourra avantageusement être de l'oxyde de potassium $K_2O$.

La quantité de promoteur pourra être comprise entre 0 et 25 % en poids du composé supporté et, typiquement, entre 1 % et 5 % en poids.

Les exemples qui suivent illustrent la mise en oeuvre de l'invention. Bien entendu, ils n'ont pas de caracrère limitatif.

## EXEMPLE 1

Cet exemple vise à comparer les propriétés de deux compositions catalytiques, l'une $C_1$ conforme à l'invention, comprenant un mélange de poudre d'une zéolithe et d'un catalyseur de fer sur support, l'autre $T_1$ constituée d'un catalyseur à base de fer supporté, utilisé seul.

Les compositions catalytiques $C_1$ et $T_1$ ont les caractéristiques suivantes :

— Catalyseur $C_1$ : Ce catalyseur comprend 80 parties en poids de zéolithe H-ZSM-5, présentant un rapport Si/Al de 60, sous la forme de particules de dimensions comprises entre 0,25 mm et 0,50 mm, et 20 parties en poids d'un catalyseur à base de fer sur support d'alumine SCS 9 (diamètre moyen des pores : 3 000 Å), sous la forme de particules de dimensions comprises entre 0,25 mm et 0,50 mm. Le composé à base de fer sur support (20 parties en poids) comprend 10% en poids d'oxyde fer $FE_2O_3$, et 5 % en poids de $K_2O$ comme promoteur, le reste étant constitué d'alumine.

– Catalyseur $T_1$ : Ce catalyseur comprend 100 parties en poids d'un catalyseur à base de fer sur support d'alumine SCS 9 (diamètre moyen des pores 3 000 Å), sous la forme de particules de dimensions comprise entre 0,25 mm et 0,50 mm. Ce composé comprend 10 % en poids d'oxyde de fer $Fe_2O_3$, avec, comme promoteur, 5 % en poids de $K_2O$, le reste étant constitué d'alumine.

Ces catalyseurs ont été utilisés pour traiter un gaz de synthèse ayant les caractéristiques suivantes:

– rapport $H_2/CO$ : 1,5,

– vitesse spatiale horaire : 2550 v/v/h,

– pression totale : 17 bar,

– pression partielle d'hydrogène : 10,2 bar,

– pression partielle de CO : 6,8 bar.

Le gaz de synthèse a été mis en contact avec chacun des catalyseurs, pendant des durées variables, à diverses températures, et les produits obtenus ont été analysés.

Le tableau I ci-après rassemble les conditions de traitement et les résultats obtenus avec chacun desdits catalyseurs.

TABLEAU I

| Catalyseur | $T_1$ | | $C_1$ | |
|---|---|---|---|---|
| Température (°C) | 300 | 370 | 300 | 350 |
| Temps de marche (h) | 8 | 42 | 5 | 27 |
| Conversion de CO (%) | 95,1 | 97,5 | 87,2 | 97,2 |
| Carbone converti en $CO_2$ (% molaire) | 59,7 | 52,9 | 56,7 | 52,2 |
| Carbone converti en hydrocarbures (% molaire) | 40,3 | 47,1 | 43,3 | 47,8 |
| Hydrocarbures gazeux $(C_1 - C_4)$ (%) | 44 | 58,3 | 58,4 | 59,7 |
| Coupe essence $(C_5-C_{10})$ (%) | 38,1 | 37 | 33,6 | 34,4 |
| Teneur en aromatiques (%) | 0,0 | 0,0 | 47,8 | 39,9 |
| Hydrocarbures à plus de 10 atomes de carbone (coupe $C_{10}+$) (%) | 17,9 | 4,7 | 8,0 | 5,9 |

Ce tableau montre que seule la composition catalytique $C_1$, conforme à l'invention, permet d'obtenir une coupe hydrocarbonée contenant peu d'hydrocarbures à plus de 10 atomes de carbone, avec une coupe essence ayant une bonne aromaticité (40 à 50 % de composés aromatiques). Ce n'est pas le cas de la composition témoin, $T_1$, qui

ne comprend pas la zéolithe H-ZSM-5, bien qu'elle contienne du fer sur un support. Ces essais confirment aussi que l'acidité de la zéolithe n'est pas affectée par la présence du fer.

Cet exemple prouve qu'en utilisant conjointement, en un seul lit, un catalyseur zéolithique et un catalyseur à base d'un composé actif en synthèse Fischer-Tropsch, déposé sur un support, il est possible de convertir un gaz de synthèse en un mélange d'hydrocarbures ayant une bonne sélectivité en coupe essence, une bonne aromaticité et contenant peu de produits lourds à plus de 10 atomes de carbone.

## EXEMPLE 2

Cet exemple vise à comparer les propriétés de deux compositions catalytiques, l'une $C_2$, conforme à l'invention, comprenant un mélange de poudres d'une zéolithe et d'un catalyseur à base de fer sur support, l'autre $T_2$, constituée d'un mélange de poudres de zéolithe et d'un catalyseur à base de fer non supporté pour la synthèse de Fischer-Tropsch.

Les compositions catalytiques $C_2$ et $T_2$ ont les caractéristiques suivantes :

- Catalyseur $C_2$ : Ce catalyseur comprend 80 parties en poids de zéolithe H-ZSM-5(60), présentant un rapport Si/Al de l'ordre de 60, et 20 parties en poids d'un catalyseur à base de fer sur support d'alumine SCS9 (diamètre moyen de pores : 3 000 Å), sous la forme de particules de dimensions comprises entre 0,25 mm et 0,50 mm. Le composé à base de fer sur support comprend 5 % en poids d'oxyde de fer pur et 0,5 % en poids de $K_2O$ comme promoteur, le reste étant constitué d'alumine.

- Catalyseur $T_2$ : Ce catalyseur comprend 75 parties en poids de zéolithe H-ZSM-5 présentant un rapport Si/Al de 117 (dimensions moyennes des particules comprises entre 0,25 mm et 0,50 mm) et 25 parties en poids d'un catalyseur à base de fer comprenant 95 % en poids d'oxyde de fer $Fe_2O_3$ et 5 % d'oxyde de potassium (dimensions moyennes des particules comprises entre 0,25 mm et 0,50 mm).

Ces catalyseurs ont été utilisés pour traiter un gaz de synthèse (rapport $H_2/CO$ : 1,5) dans les conditions suivantes :

- vitesse spatiale horaire   :   1 750 v/v/h,
- pression totale   :   17 bar,
- pression partielle $H_2$   :   10,2 bar,
- pression partielle CO   :   6,8 bar.

Le gaz de synthèse a été mis en contact pendant des durées variables avec chacun des catalyseurs, à diverses températures, et les produits obtenus ont été analysés.

Le tableau II ci-après rassemble les conditions de traitement et les résultats obtenus avec les deux catalyseurs.

0148048

TABLEAU II

| Catalyseur | $C_2$ | | | $T_2$ | |
|---|---|---|---|---|---|
| Température (°C) | 300 | 350 | 400 | 300 | 350 |
| Temps de marche (h) | 4,5 | 8,0 | 11,0 | 26,0 | 30,0 |
| Conversion de CO (%) | 23,0 | 73,9 | 78,6 | 67,5 | 92,5 |
| $CO_2$ obtenu (% molaire par rapport au carbone) | 48,0 | 48,5 | 52,4 | 43,0 | 28,0 |
| Hydrocarbures obtenus (% molaire par rapport au carbone) | 52,0 | 51,5 | 47,6 | 57,0 | 72,0 |
| Hydrocarbures gazeux $(C_1 - C_4)$ % | 58,6 | 64,2 | 75,6 | 46,9 | 39,8 |
| Coupe essence $(C_5-C_{10})$ % | 41,4 | 35,8 | 24,4 | 28,8 | 32,1 |
| Teneur en aromatiques % | 55,5 | 62,7 | 58,7 | 4,1 | 17,3 |
| Hydrocarbures à plus de 10 atomes de carbone (coupe $C_{10}^+$ en % ) | 0,0 | 0,0 | 0,0 | 24,7 | 28,1 |

Ce tableau montre que seule la composition catalytique $C_2$ conforme à l'invention permet d'obtenir une coupe hydrocarbonée ne contenant pas ou que peu d'hydrocarbures à plus de 10 atomes en carbone, avec une coupe "essence" ayant une bonne aromaticité (55 à 63 % de composés aromatiques). Ce n'est pas le cas de la composition

témoin $T_2$, bien que celle-ci comprenne aussi une proportion importante de zéolithe H-ZSM-5, dont les caractéristiques (acidité et réseau cristallin) favorisent l'obtention de ces résultats.

La composition $C_2$ conforme à l'invention semble avoir une activité plus faible que celle de la composition $T_2$, mais on notera que cette dernière contient beaucoup plus de fer que la composition $C_2$.

L'absence quasi-totale de coupe $C_{10}+$, lorsque l'on utilise la composition $C_2$, semble montrer que cette coupe est craquée en produits légers (fraction $C_1-C_4$) sur le catalyseur au fer supporté, ce qui est typique d'un catalyseur acide. Ceci confirme donc que, dans le cas de la composition catalytique $C_2$ de l'invention, l'acidité de la zéolithe n'est pas affectée par la présence du fer. Au contraire, dans le cas des catalyseurs au fer non supportés, le fer et le potassium ont un effet conjugué d'empoisonnement des sites acides des zéolithes H-ZSM-5.

Cet exemple prouve donc qu'en utilisant conjointement en un seul lit un catalyseur zéolithique et un catalyseur à base de fer sur support d'alumine, il est possible de convertir un gaz de synthèse en un mélange d'hydrocarbures ayant une bonne sélectivité en coupe essence, une bonne aromaticité et dépourvu de produits lourds à plus de 10 atomes de carbone.

EXEMPLE 3

Cet exemple vise à comparer les propriétés de trois compositions catalytiques, l'une, $C_3$, conforme à l'invention, comprenant un mélange de poudres d'une zéolithe et d'un catalyseur à base de cobalt (et d'un promoteur) sur un support, une autre, $C_3'$, constituée d'un mélange de poudres d'une zéolithe et d'un catalyseur à base de cobalt (sans promoteur) sur un support, et la dernière, $T_3$, constituée d'un catalyseur à base de cobalt supporté, utilisé seul.

Les compositions catalytiques $C_3$, $C_3'$ et $T_3$ ont les caractéristiques suivantes :

- Catalyseur $C_3$ : ce catalyseur comprend 80 parties en poids de zéolithe H-ZSM-5, présentant un rapport Si/Al de 40, sous la forme de particules de dimensions comprises entre 0,10 mm et 0,30 mm. Le composé à base de cobalt (20 parties en poids) sur support comprend en lui-même 5 % en poids de cobalt Co, 1 % en poids d'oxyde de thorium $ThO_2$ et 1 % en poids d'oxyde de magnésium MgO comme promoteurs, le reste étant constitué d'alumine.

- Catalyseur $C_3'$ : ce catalyseur comprend 80 parties en poids de zéolithe H-ZSM-5, présentant un rapport Si/Al de 40, sous la forme de particules de dimensions comprises entre 0,10 et 0,30 mm. Le composé à base de cobalt (20 parties en poids) sur support comprend en lui-même 5 % en poids de cobalt Co, le reste étant constitué d'alumine.

- Catalyseur $T_3$ : ce catalyseur est constitué d'un composé à base de cobalt sur support d'alumine, comprenant en lui-même 5 % en poids de cobalt Co, le reste étant constitué d'alumine.

Ces catalyseurs ont été utilisés pour traiter un gaz de synthèse ayant les caractéristiques suivantes :

- rapport $H_2/CO$ : 1,5,
- vitesse spatiale horaire : 2 500 v/v/h,
- pression totale : 17 bar,
- pression partielle d'oxygène : 10,2 bar,
- pression partielle de CO : 6,8.

Ce gaz de synthèse a été mis en contact avec chacun des catalyseurs décrits ci-dessus, pendant des durées variables, à diverses températures, et les produits obtenus été analysés.

Le tableau III donné ci-après rassemble les conditions de traitement et les résultats obtenus avec chacun desdits catalyseurs.

TABLEAU III

| Catalyseur | $T_3$ | $C_3'$ | $C_3$ |
|---|---|---|---|
| Température (°C) | 300 | 300 | 300 |
| Temps de marche (h) | 11 | 7 | 7 |
| Conversion de CO (%) | 58,8 | 64,1 | 62,6 |
| $CO_2$ obtenu (% molaire) | 30,0 | 27 | 30,4 |
| Hydrocarbures obtenus (% molaire par rapport au carbone) | 70,0 | 73 | 69,6 |
| Hydrocarbures gazeux $(C_1 - C_4)$ % | 55,3 | 77,3 | 68,2 |
| Coupe essence $(C_5-C_{10})$ % | 32,4 | 22,5 | 30,7 |
| Teneur en aromatiques | 0,00 | 23,3 | 40,7 |
| Hydrocarbures à plus de 10 atomes de carbone (coupe $C_{10}^+$ en %) | 12,3 | 0,2 | 1,1 |

Cet exemple montre que seules les compositions catalytiques $C_3$ et $C_3'$, conformes à l'invention, permettent d'obtenir une coupe hydrocarbonée "essence" ayant une bonne aromaticité.

REVENDICATIONS

1.- Nouvelle composition catalytique comprenant un mélange intime de poudres d'au moins un composé à base de fer ou de cobalt actif dans la synthèse de Fischer-Tropsch et d'une zéolithe, cette composition étant caractérisée en ce que ledit composé repose sur un support.

2.- Composition catalytique selon la revendication 1, caractérisée en ce qu'elle comprend entre 5 et 98 % en poids de zéolithe et entre 2 et 95 % en poids de composé à base de fer ou de cobalt.

3.- Composition catalytique selon l'une des revendications 1 et 2, caractérisée en ce que la zéolithe est choisie dans le groupe comprenant les zéolithes du type ZSM-5, ZSM-11, ZSM-12, ZSM-34, ZSM-35, ZSM-38, ZSM-48, zéolithe PHI et zéolithe BETA et tout autre composé métallo-silicate cristallin microporeux.

4.- Composition catalytique selon l'une des revendications 1 et 2, caractérisée en ce que la zéolithe est du type ZSM-5.

5.- Composition catalytique selon l'une des revendications 1 à 4, caractérisée en ce que le support du composé est choisi dans le groupe constitué par : alumine, silice, zéolithe, argile, argile intercalée et plus généralement tous oxydes de métaux, ou un mélange de plusieurs ou de tous ces composés.

6.- Composition catalytique selon l'une des revendications 1 à 5, caractérisée en ce que le support est constitué d'alumine présentant une dimension moyenne de pores comprise entre 3 Å et 20 000 Å.

7.- Composition catalytique selon l'une des revendications 1 à 6, caractérisée en ce que ledit composé contient du fer ou du cobalt présent sous la forme métallique ou d'oxyde, éventuellement combiné avec des promoteurs choisis dans le groupe formé par le cuivre, le fer, l'alumine, l'oxyde de chrome, des composés alcalins ou alcalino-terreux, le ruthénium et des terres rares sous forme oxydée ou réduite.

8.- Composition catalytique selon l'une des revendications 1 à 7, caractérisée en ce que ledit composé contient également un promoteur constitué d'oxyde de potassium $K_2O$.

9.- Application de la composition catalytique selon l'une des revendications 1 à 8, à la conversion de gaz de synthèse en un mélange d'hydrocarbures à aromaticité élevée, par passage dudit gaz de synthèse, dans des conditions de conversion, sur un lit de ladite composition catalytique.

10.- Application selon la revendication 9, caractérisée en ce que ladite conversion est conduite à une température comprise entre 200 et 500°C, sous une pression de 1 à 100 bar, à une vitesse spatiale horaire de 100 à 10 000 v/v/h et avec un rapport $H_2/CO$ de la charge traitée compris entre 0,5 et 3,5.

Office européen
des brevets

RAPPORT DE RECHERCHE EUROPÉEN

**0148048**
Numero de la demande

EP 84 40 2377

## DOCUMENTS CONSIDERES COMME PERTINENTS

| Categorie | Citation du document avec indication, en cas de besoin, des parties pertinentes | Revendication concernée | CLASSEMENT DE LA DEMANDE (Int Cl 4) |
|---|---|---|---|
| A | DE-A-3 236 093 (TOYO ENGINEERING) * Revendication 1 * | 1-10 | C 07 C 1/04 B 01 J 29/06 B 01 J 23/74 |
| A | US-A-4 207 208 (MOBIL OIL) * Colonne 1, lignes 20-25, 47-68; colonne 2, lignes 1-3; colonne 3, lignes 36-68; colonne 4, lignes 36-41; colonne 5, lignes 33-35; exemples 1-3,8 * | 1-5,7 9,10 | |
| A | US-A-3 620 963 (CHEVRON) * Résumé; colonne 2, lignes 64-70; colonne 6, lignes 50-60 * | 1-8 | |
| A | EP-A-0 029 255 (UNIVERSITY OF DELAWARE) * Revendications 1,2 * | 1-10 | DOMAINES TECHNIQUES RECHERCHES (Int. Cl.4) |
| A | DE-C- 763 233 (METALLGESELLSCHAFT) * Revendication 1 * | 7 | C 07 C B 01 J |
| A | DE-B-1 002 746 (METALLGESELLSCHAFT) * Revendications 1,3 * | 7 | |
| A | US-A-4 207 248 (MOBIL OIL) * Colonne 7, lignes 3-11 * | 10 | |

Le present rapport de recherche a été établi pour toutes les revendications

| Lieu de la recherche | Date d'achèvement de la recherche | Examinateur |
|---|---|---|
| LA HAYE | 21-02-1985 | SALA P.C. |

CATEGORIE DES DOCUMENTS CITES

X : particulièrement pertinent à lui seul
Y : particulièrement pertinent en combinaison avec un autre document de la même categorie
A : arriere-plan technologique
O : divulgation non-écrite
P : document intercalaire

T : théorie ou principe à la base de l'invention
E : document de brevet anterieur, mais publié à la date de depôt ou apres cette date
D : cité dans la demande
L : cité pour d'autres raisons

& : membre de la même famille, document correspondant

OEB Form 1503 03 82